# EUROPEAN PATENT SPECIFICATION

(11) **EP 0 422 217 B1**
(45) Date of publication and mention of the grant of the patent: **28.01.1998**
(21) Application number: 90908084.8
(22) Date of filing: 18.04.1990
(51) Int. Cl.: C12P 21/00, C12N 15/00, C12N 1/20

(54) **HUMAN RECOMBINANT PLACENTAL RIBONUCLEASE INHIBITOR AND METHOD OF PRODUCTION**
MENSCHLICHER REKOMBINANTER PLAZENTALER RIBONUKLEASE-INHIBITOR UND VERFAHREN ZUR HERSTELLUNG
INHIBITEUR DE RIBONUCLEASE PLACENTAIRE HUMAIN DE RECOMBINAISON ET SON PROCEDE DE PRODUCTION

(30) Priority: 24.04.1989 US 342362
(43) Date of publication of application: 17.04.1991
(73) Proprietor: PROMEGA CORPORATION, Madison, WI 53711-5399 (US)
(72) Inventor: LEWIS, Martin, Kendall, Madison, WI 53704 (US); SHULTZ, John, William, Verona, WI 53593 (US)
(74) Representative: Ellis-Jones, Patrick George Armine
(86) International application number: US9002122
(87) International publication number: WO9012881

(56) References cited:
- EP-A- 0 291 686
- EMBO JOURNAL vol. 7, no. 13, 20 December 1988, IRL PRESS LIM., OXFORD, ENGL.; pages 4151 - 4156; R. SCHNEIDER ET AL.: 'The primary straucture of human ribonuclease/angiogenin inhibitor (RAI) discloses a novel highly diversified protein superfamily with a common repetitive module'
- DNA CLONING, A PRACTICAL APPROACH, EDITED BY GLOVER , IRL PRESS vol. III, 1987, OXFORD, ENGL. pages 59 - 88; F.A.O. MARSTON: 'The purification of eucaryotic polypeptides expressed in Escherichia coli'
- Biochemistry, Volume 27, Number 23, 15 November 1988, pages 8545-8553, "Primary Structure of Human Placental Ribonuclease Inhibitor", see the Sequence of figure 2 page 8547.
- Proceedings of the National Academy of Science, Volume 84, April 1987, pages 2238-2241 "Human Placental Ribonuclease Inhibitor Abolishes Both Angiogenic and Ribonucleolytic Activities of Angiogenin"
- Biochemistry, Volume 28, Number 17, July 1989, page 7138 LEE et al, "Primary Structure of Human Placental Ribonuclease Inhibitor", see the Abstract
- Biochemical and Biophysical Research Communications, Volume 101, Number 2, 30 July 1981, pages 396-403, "Effect of Human Placental Ribonuclease Inhibitor in Cell-Free Ribosomal RNA Synthesis" see the entire document, especially Abstract.
- Biochemical and Biophysical Rescarca Communications, Volume 160, Number 1 14 April 1989 pages 115-122 LEE et al, "Expression of Human Placental Ribonuclease Inhibitor in Escherichia Coli

## Description

### BACKGROUND OF THE INVENTION

### 1. Field of the Invention

The present invention relates to recombinant DNA technology. More specifically, it involves the creation of a cDNA genetic sequence encoding for human recombinant placental ribonuclease inhibitor, a vector containing this gene, and a host containing the cDNA gene.

### 2. Description of the Prior Art

Through the development of recombinant DNA techniques, it has become fairly straightforward to clone DNA sequences from essentially any organism into plasmid or viral vectors for propagation and amplification in a foreign host. In this form the DNA can be studied with regard to its sequence, structure, coding capacity or other properties. It can also be used for a variety of applications such as detection of complementary sequences in samples, generation of altered forms of a gene product, modulation of organismal function through insertion into new organisms, etc.

The advent of recombinant DNA (rDNA) technology has also resulted in the isolation of coding sequences of complementary DNA (cDNA) using antibody probes. Young, R.A. and R.W. Davis, Proc. Natl. Acad. Sci. U.S.A., 80, 1194-1198 (1983). This system incorporates features that maximize the ability to detect antigenic determinants (epitopes) on polypeptides expressed from the DNA of any source. Successful isolation of genomic or cDNA sequences using this system requires both a cDNA library and an antibody probe of adequate quality. Mierendorf, Robert C., et al., "Gene Isolation by Screening [Lambda] gtll Libraries with Antibodies," Guide to Molecular Cloning Techniques, 152, 458-469 (1987). The application of this technology has led to the isolation of the gene coding for a particular protein to which an antibody has been raised. Even so, gene isolation using this technology is by no means assured. For example, in some cases the construct that one seeks may prove to be lethal to the host cell, most generally E.coli. In other cases, the isolation of the gene is made difficult by producing antibodies mainly against the carbohydrate portion of the target protein, especially when the carbohydrate is not added onto the protein when synthesized within the E.coli cell. In situations like this, the antibody is not useful for detection. A further complication is the appearance of weak positives on the immunoscreening filter. The weak positives presumably are proteins which are recognized by antibodies against contaminants in the immunizing protein preparation. Alternatively, the weak positives may be other proteins with shared immunogenicity to the protein of interest.

Final proof that the target protein has been cloned will depend upon achieving activity of the recombinant protein synthesized in E.coli. This is sometimes not possible since many eukaryotic proteins are synthesized in a misfolded, insoluble and inactive form in E. coli. Hoess, A., et al., "Recovery of Soluble, Biologically Active Recombinant Proteins from Total Bacterial Lysates Using Ion Exchange Resin," Biotechnology, 6, 1214-1217 (1988). In some cases the inactive, insoluble protein can be solubilized and refolded into an active form. However, the determination of the conditions essential for the efficient recovery of the active form of a protein requires significant experimentation to optimize many parameters that can affect the refolding process. See Marston, F.A.O., "The Purification of Eukaryotic Polypeptides Expressed in Escherichia coli," DNA Cloning, Vol. III. A Practical Approach, Chap. 4, D.M. Glover, Ed., IRL Press, 1987, and the references cited therein. Thus, the testing of a cloned gene encoding a particular protein depends on many factors that cannot be predicted before experimentation is initiated.

It is generally thought that the isolation of a cDNA clone, i.e., a double-stranded DNA which corresponds to the information present in the RNA for the protein, first involves sufficiently purifying enough of the protein of interest and developing an antibody against that protein in an appropriate animal, such as a rabbit. The next step is to use the antibody as an immunoprobe to screen a cDNA library of the tissue where such protein is expressed. cDNA libraries are most frequently constructed in bacteriophage lambda vectors, and particularly the bacteriophage vector lambda gtll when an antibody probe is available for screening. This is because lambda gtll is an expression vector, meaning that a fusion protein is formed between E.coli beta galactosidase, a natural E. coli enzyme, and the protein from the cDNA inserts. Jendrisak, Jerry, et al. (1987) "Cloning cDNA into [Lambda] gt10 and [Lambda] gtll," Guide to Molecular Cloning Techniques, 152, 359-371 (1987). The gene for the protein of interest, present in the form of a DNA copy of the RNA, is also present in the lambda gtll under the control of an E.coli promoter.

When the recombinant bacteriophage encoding for the desired protein infects E.coli cells, some of the recombinant fusion protein is produced and released from the cells due to cell lysis in the bacteriophage plaque. This protein is picked up on a nitrocellulose filter, and the protein is detected with the antibody against the protein of interest. The screening procedure is repeated until homogeneous phage plaques carrying the foreign gene are produced. The gene is then ready for subcloning into a plasmid, a small circular form of DNA that can replicate independently of the DNA in the genome, and for engineering for expression.

This technology has been applied to human placental ribonuclease inhibitor (PRI). Blackburn, Peter, et al., "Ribonuclease Inhibitor from Human Placenta," The Journal of Biological Chemistry, 252/16, 5904-5910 (1977). Blackburn, et al. disclose the preparation of a soluble PRI, which had been purified 4,000 fold by a combination of ion exchange and affinity chromatography. PRI was found to be an acidic protein of molecular weight near 50,000.

Natural PRI is a protein isolated from human placenta which specifically inhibits ribonucleases (RNases), an enzyme that catalyzes the breakdown of RNA. It functions by binding tightly to a wide spectrum of RNases and can be very useful wherever strong RNase inhibition may be essential to protect RNA. PRI is a protein of interest, having great promise for utility in research and other purposes. Although the physiological role of the protein has not yet been established, recent data have suggested that the in vivo role of the protein may be the regulation of blood vessel development. Shapiro, Robert and Bert L. Vallee, "Human Placental Ribonuclease Inhibitor Abolishes Both Angiogenic and Ribonucleolytic Activities of Angiogenin," Proc. Natl. Acad. Sci. USA, 84, 2238-2241 (1987). Shapiro and Vallee established the relationship between PRI and angiogenin. The results of their experiments suggest that PRI and related inhibitors may participate in the in vivo regulation of angiogenin, a blood vessel-inducing protein from HT-29 human:adenocarcinoma cells. Human PRI has been found to abolish both the biological and enzymatic activities of angiogenin, thus further confirming that the angiogenin/PRI interaction is functionally significant. Because of the possible participation of angiogenin in creating states of pathological vascularization such as occur in a variety of diseases including tumor metastasis, diabetic retinopathy and rheumatoid arthritis, the tight interaction with and inhibition of angiogenin by PRI could potentially be a useful therapeutic route to treat these diseases. Thus, PRI may have important mechanistic, physiologic, pharmacologic and/or therapeutic implications. Still other functions for this protein are likely to be discovered.

Lee and Vallee (Biochemical & Biophysical Research Commun, 1989, vol 160, pp 115 - 120 discloses the expression of human PRI in E. Coli and isolation from the host cells

To study the role of the protein PRI in animals requires a pure source which is free from the impurities that may be present in preparations from the human placental source. These impurities are mammalian proteins, possibly prions, and mammalian DNA, some of which may be from viral sources such as HIV and hepatitis virus. Unfortunately, it is relatively expensive to extract a sufficient amount of the purified protein from natural sources. Thus, there is a need for a source of large, less expensive quantities of human PRI which are essentially free of impurities.

### SUMMARY OF THE INVENTION

In accordance with the present invention, it is now possible to obtain usable quantities of a cloned, active gene product of human PRI which is free from contaminants. The procedure involves the steps of isolating the natural gene encoding for human PRI starting with a sample of human PRI, screening a DNA library for the presence of a cloned gene, forming a human PRI plasmid construct, introducing the plasmid construct into a host cell, and isolating the human PRI protein by cell lysis followed by centifugation to recover the human PRI.

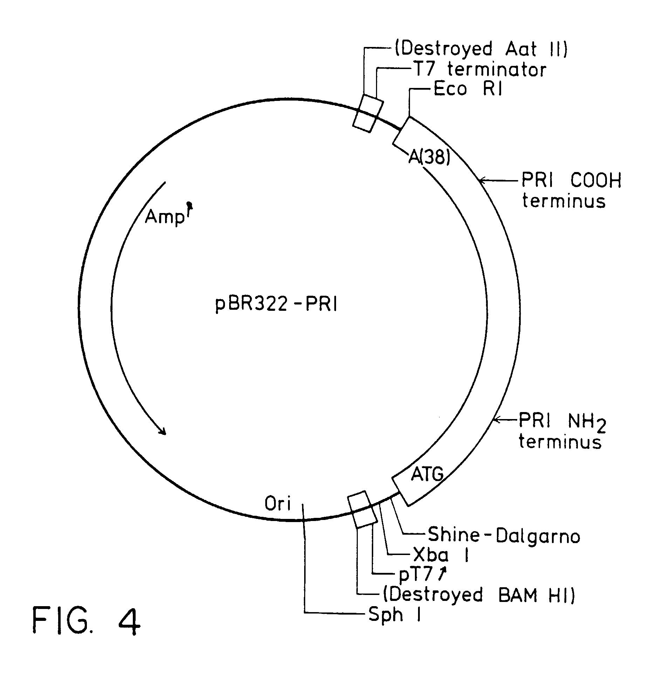

The present invention provides a plasmid which is replicatable in E. coli and comprises a DNA sequence encoding human placental ribonuclease inhibitor in a DNA segment which is the same as the segment of plasmid pBR322-PRI that corresponds to the sequence encoding human placental ribonuclease inhibitor that lies between the destroyed BamHI and destroyed AatII sites as shown in Figure 4.

The invention also provides an E. coli strain which comprises an inducible T7 RNA polymerase gene and is transformed with a plasmid of the invention, and to a method of obtaining cloned active human PRI, which method comprises culturing an E. coli strain of the invention and isolating, in active form, human PRI thus produced.

The present invention is also directed to a method of solubilizing, and activating human PRI from inclusion bodies produced in E. coli, which method comprises:
combining the inclusion bodies with a first buffer solution comprising urea at a concentration no less than 4M over a period of time no greater than thirty minutes;
combining the solution of inactive inhibitor with a second buffer solution at a rate of at least 20 parts second buffer to 1 part of the solution of inactive inhibitor, said second buffer having a pH between 6.5 and 8.5, comprising DTT at a concentration of not less than 0.5 mM, and EDTA; and
allowing sufficient time for the inhibitor to refold to an active state.

In addition, in accordance with the invention, the gene sequence coding for recombinant human PRI has been determined.

Further objects, features and advantages of the invention will be apparent from the following detailed description of the invention and the illustrative drawings.

### BRIEF DESCRIPTION OF THE DRAWINGS

Fig. 1 is an illustration of a partial restriction site and functional map of the plasmid pGEM®-7Zf(+).

Fig. 2 is an illustration of a partial restriction site and functional map of the plasmid pGEM®-7Zf(+) having the gene sequence encoding for PRI inserted therein.

Figs. 3-3C illustrate the nucleotide sequence of the cloned gene coding for human PRI.

Fig. 4 is an illustration of a partial restriction site and function map of a pBR322 plasmid having the gene sequence encoding for PRI inserted therein and engineered for expression.

### DETAILED DESCRIPTION OF THE INVENTION

The present invention is directed to a the production of human PRI by expressing the product of the gene encoding this, and purifying and refolding the recombinant human PRI. The PRI may be obtained from a host cell which carries a plasmid containing the gene for the PRI protein. The DNA sequence of the gene for human PRI is also described.

An important aspect of the present invention involves the isolation of the inactive recombinant PRI, the solubilization of the recombinant PRI and the activation of the solubilized recombinant PRI.

A variety of vectors replicatable in E. coli can be used to carry the gene coding for recombinant PRI into host cells including pBR322 plasmids,

Host cells used in the invention are E. coli cells. The host cell, in combination with the vector and the foreign gene sequence coding for PRI, which is subject to the control of the promoter, make up the recombinant host cell capable of expressing human PRI.

The recombinant PRI is characterized by a number of properties, among them:
a) molecular weight: 51,000 daltons
b) type of inhibition: non-competitive

Additionally recombinant PRI is distinguished from PRI from a natural source by a number of characteristics. For example, unlike the PRI from a natural source, the N-terminal amino acid of the recombinant PRI is specified. Additionally and unlike the natural PRI, the recombinant PRI is not blocked and not acetylated. Further, the gene coding for recombinant PRI is free of mammalian DNA, prions and other potentially detrimental material such as, for example, HIV.

The inactive recombinant PRI is present as an inclusion body in a host cell. Typically, the inclusion body is a densely packed, granular protein mass. In order to isolate the PRI from the host cell, the cell membrane must be disrupted. Although there are several known cell lysis techniques available, the preferred method involves the use of an appropriate amount of lysozyme followed by centrifugation and a second treatment with sodium dodecyl sulfate (SDS). This process yields about 0.1% soluble active PRI in solution and about 99.9% insoluble, inactive PRI. The insoluble protein is removed in the form of crude, inactive protein by centrifugation. The protein is further purified by buffer suspension/centrifugation techniques.

After the inactive PRI protein has been isolated and purified, it is next solubilized, i.e., placed into solution. Solubilization is initiated in a buffer solution containing urea intended to separate the polypeptide chains from each other in a solution. Prior to use, the urea solution is preferably centrifuged to remove unwanted debris and cell matter. The solubilization step should take place over a relatively short span of time, i.e., no more than 30 minutes. It has been discovered that there is an inverse correlation between the length of time that the inactive PRI associates with the chemical agent and the final yield of active PRI.

The activation step involves the dilution of the solution containing the PRI in a buffer solution for a time sufficient to allow the PRI to refold to an active state. Several factors are important for the activation state. For example, the dilution step should take place relatively quickly, i.e., within 2 minutes, preferably within 30 seconds. The buffer solution should have a pH in the range of about 6.5 and about 8.5, preferably about 7.5. It is also helpful to provide the buffer solution with a water active agent such as glycerol and sucrose. It has been discovered that the presence of a sufficient quantity of water active agent in the buffer solution enhances the PRI protein refolding ability. It has also been found that a dilution rate of about 1 part PRI solution to about 100 parts buffer solution is optimal to assist in refolding the PRI.

After the solution has been diluted, it should be allowed to remain undisturbed for a period of time not less than 8 hours, and preferably between 8 and 18 hours at a temperature between about 10° C and 25° C. Under these conditions, the PRI protein should refold such that about 7% of PRI molecules will become active.

The following examples are provided as illustrative of the methods for producing a cloned gene encoding for human PRI and for producing active recombinant human PRI.

### Example 1

### Isolation of a Lambda gt11 Clone Containing a Gene for Human PRI

Before the gene for human PRI can be cloned, a substantially pure natural human PRI protein must be obtained. The human PRI protein is prepared according to the process of Blackburn, et al. (supra) which describes the purification process. Briefly, a soluble ribonuclease inhibitor from the human placenta can be purified 4,000 fold by a combination of ion exchange and affinity chromotography described in Blackburn, et al. (supra).

PRI protein from the natural source was purified to apparent homogeneity according to the method described in Blackburn, et al. (supra). The protein was further purified by binding to and eluting from DEAE Sepharose* CL-6B by gradient elution, a process which removes a functional contaminant whose presence may not be obvious from gel analysis.
*Sepharose is believed to be a registered trade march in one or more of the designated states.

A laboratory animal, a New Zealand white rabbit, was immunized with 1 mg. of the purified PRI protein, followed by subsequent booster injections, and bled at 10 weeks. A commercial cDNA library (Clontech, Palo Alto, California) of human placenta in the bacteriophage lambda gtll, was screened according to the procedure listed in the Protoblot Immunoscreening Manual (Promega Corporation, 1987). Briefly, the library was plated on E.coli Y1090 (r minus) at a density of 50,000 plaques per plate and 10 plates were screened for a total of 500,000 plaques. A plaque giving a strong positive signal was identified.

The plates were overlayered with nitrocellulose filter discs which had been soaked in isopropyl-beta-D-thiogalactopyranoside (IPTG). Following adsorption of the proteins to the filters, the filters were lifted and the rest of the protein binding sites on the filters were blocked by incubation in a buffer containing 1% v/v bovine serum albumin (BSA). The filters were then exposed to a 1,000 fold dilution of the rabbit antibody, diluted in buffer and shaken gently at room temperature for approximately 30 minutes. The filters were then washed 3 times in buffer and exposed to a 1:7500 dilution of commercially available goat anti-rabbit IgG alkaline phosphatase conjugate (Promega Corporation). This "second antibody" detects the presence of a first antibody bound to the PRI fusion protein on the filter. Following a 30 minute room temperature incubation in the presence of the second antibody, the filters were then washed 3 times in buffer. A substrate solution for alkaline phosphatase was then added and the color development allowed to proceed. One plaque giving a strong positive signal was observed in a single run of screening 500,000 plaques. The plaque producing the strong positive was then purified to homogeneity by cutting out the plaque and replating until the particular phage was pure.

The DNA from the recombinant lambda phage carrying a putative PRI gene was purified using a commercially available phage absorbent (Lambdasorb®, Promega Corporation) by immunoprecipitation. Restriction analysis of the phage DNA indicated that a single 1.6 kilobase (kb) insert was present. This insert could be removed from the phage DNA by digestion with the enzyme Eco R1. Since the PRI protein would require a 1.4 kb gene in order to encode for it, this insert was of an appropriate size to carry the entire PRI gene. In general, eukaryotic messenger RNA's are longer than just required to carry a protein coding sequence, being augmented by a 5' untranslated region and a 3' untranslated region, as well as a region carrying a poly A tract.

### Example 2

### Subcloning the Gene Containing the PRI Insert Into a Plasmid

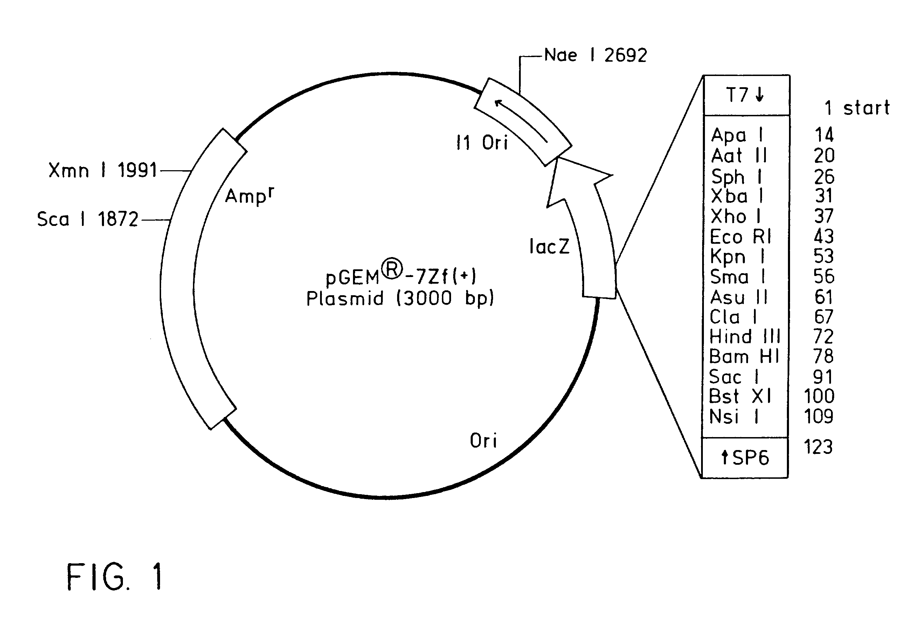

In Example 1, the PRI gene was removed from the lambda gtll on a single Eco R1 fragment. Therefore, it was decided to directly subclone the fragment into the plasmid pGEM®-7Zf(+) (Promega Corporation). Reference is made to Figure 1 for an illustration of the plasmid pGEM®-7Zf(+). By subcloning the fragment into the pGEM®-7Zf(+) plasmid, expression of the correct reading frame of the insert could be obtained.

The reading frame at the Eco R1 site of the pGEM®-7Zf(+) plasmid is the same as the Eco R1 site of the bacteriophage lambda gtll. Thus, if the coding sequence of the cDNA is expressed in lambda gtll, it will also be expressed in the pGEM®-7Zf(+) plasmid.

When the fragment is inserted into the vector in correct orientation, the promoter on the plasmid, i.e., the lac promoter, drives the expression of the PRI insert. Attached to the PRI protein is a protein segment derived from the first few amino acids of beta-galactosidase, as well as extra amino acids coded by the multiple cloning region in the pGEM®-7Zf(+) plasmid vector and any 5' non-coding regions present in the insert before the ATG of the PRI protein.

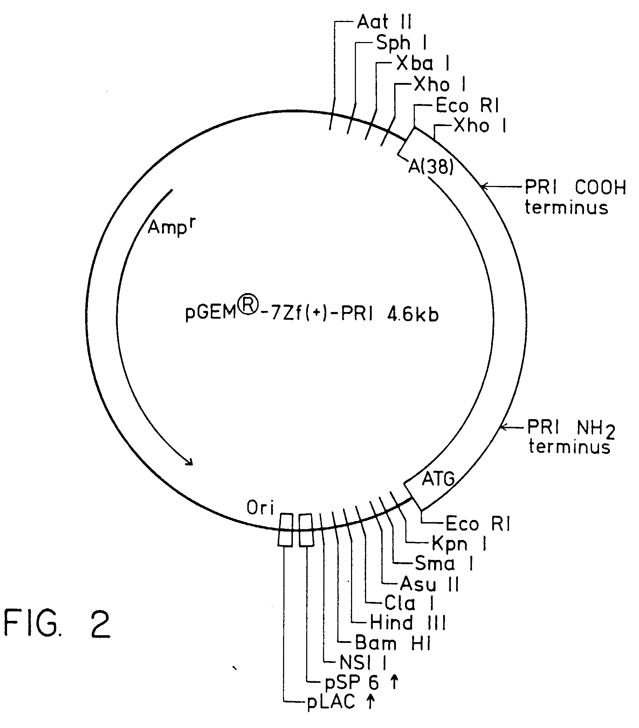

The bacteriophage lambda gtll carrying the Eco R1 PRI insert was cut with Eco R1 and subcloned into the pGEM®-7Zf(+) plasmid by standard cloning techniques as described in Maniatis, et al., Molecular Cloning. A Laboratory Manual, (1982) Cold Spring Harbor, Laboratory Press, Cold Spring Harbor, New York. Reference is made to Fig. 2 for a map of this recombinant plasmid.

### Example 3

### Expression of PRI Insert in pGEM®-7Zf(+1 Plasmid

When transferred into the pGEM®-7Zf(+) plasmid, the insert is in a frame for expression, i.e., the expression frame at the Eco R1 site in this plasmid is the same frame as the expression frame at the Eco R1 site in the lambda gtll. Therefore, the PRI fusion protein could be directly produced in E.coli. Following induction of expression by inducing the lac promoter on the pGEM®-7Zf(+) plasmid, a fusion protein with an apparent molecular weight of about 60,000 daltons was detected by Western blot analysis (Towbin, H., et al., Proc. Natl. Acad. Sci. U.S.A., 76, 4350 (1979)) utilizing primary rabbit antibody against natural PRI as the probe.

It was expected that the fusion protein produced would be somewhat larger than the native PRI. This was, in fact, observed to be the case and is consistent with the hypothesis that the entire coding region of PRI was present on the Eco R1 insert.

### Example 4

### Production of PRI Protein in a Rabbit Reticulocyte Lysate

Following the cloning of the PRI insert into the pGEM®-7Zf(+) plasmid, the vector was now available for the synthesis of RNA from the insert in vitro using the SP6 promoter on the vector. RNA was synthesized from this promoter and used to program a rabbit reticulocyte lysate. A 51,000 dalton protein was synthesized which had the size of natural PRI protein. Again, this result indicates that the entire PRI coding sequence is contained on the clone.

### Example 5

### Sequencing of the Insert in the pGEM®-7Zf(+) Plasmid

An initial sequencing run of the 5' end and the 3' end of the PRI gene insert was performed on the clone in the pGEM®-7Zf(+) plasmid. The sequencing data indicated the presence of an ATG codon several nucleotides downstream from the Eco R1 cloning site and the presence of a poly A tail 36 residues in length at the 3' end of the cloned gene. From the size of the fusion protein produced according to Example 4, it was possible to conclude that the logical starting point for the PRI protein coding sequence was the first ATG codon discovered in the sequencing clone.

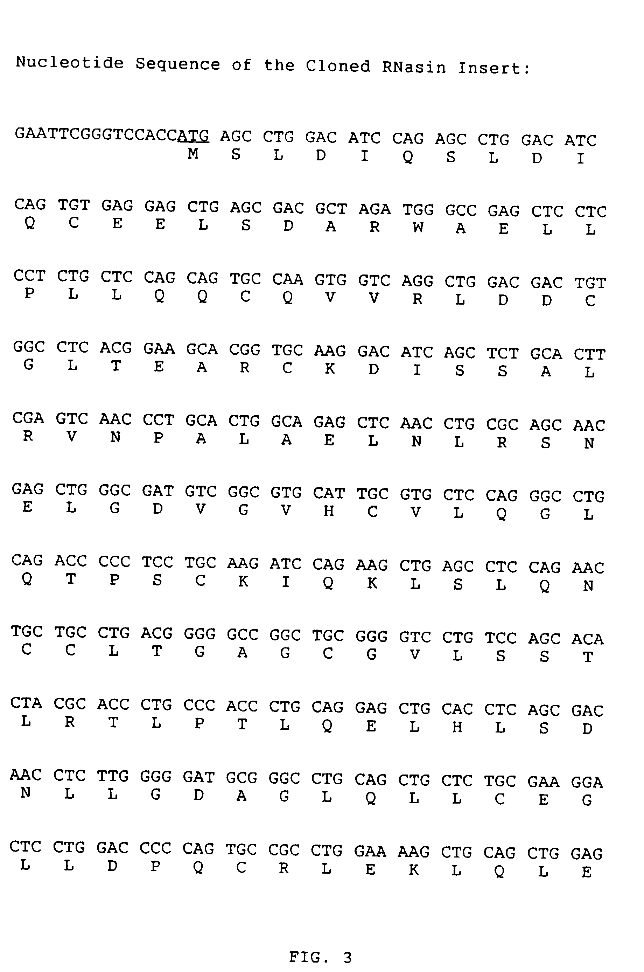

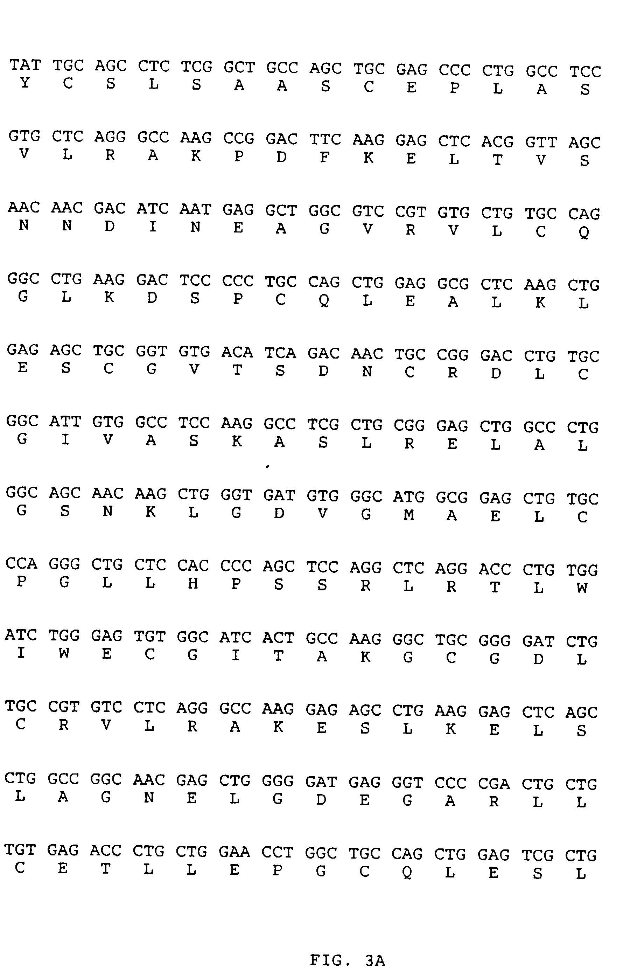

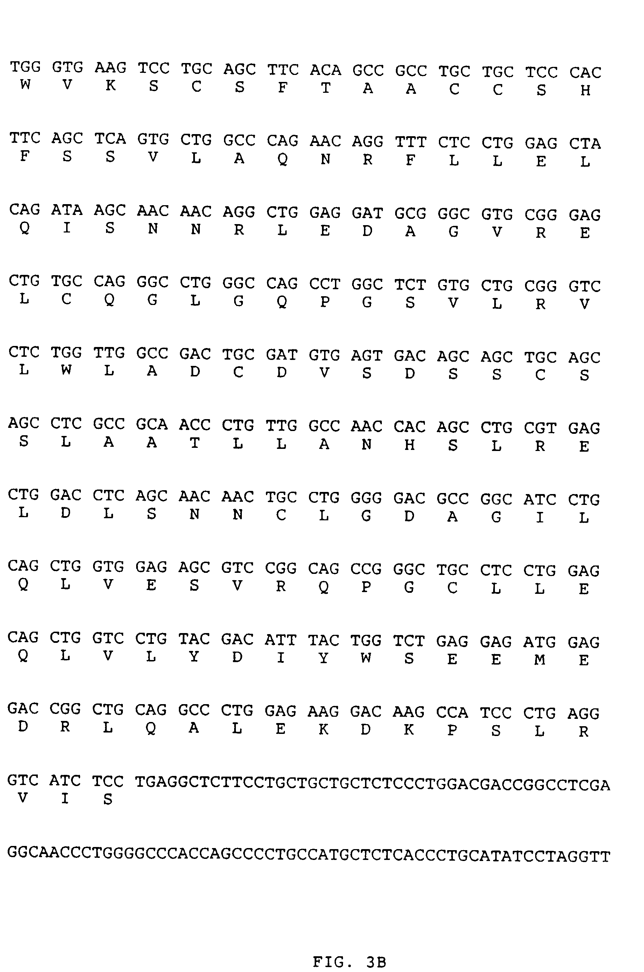

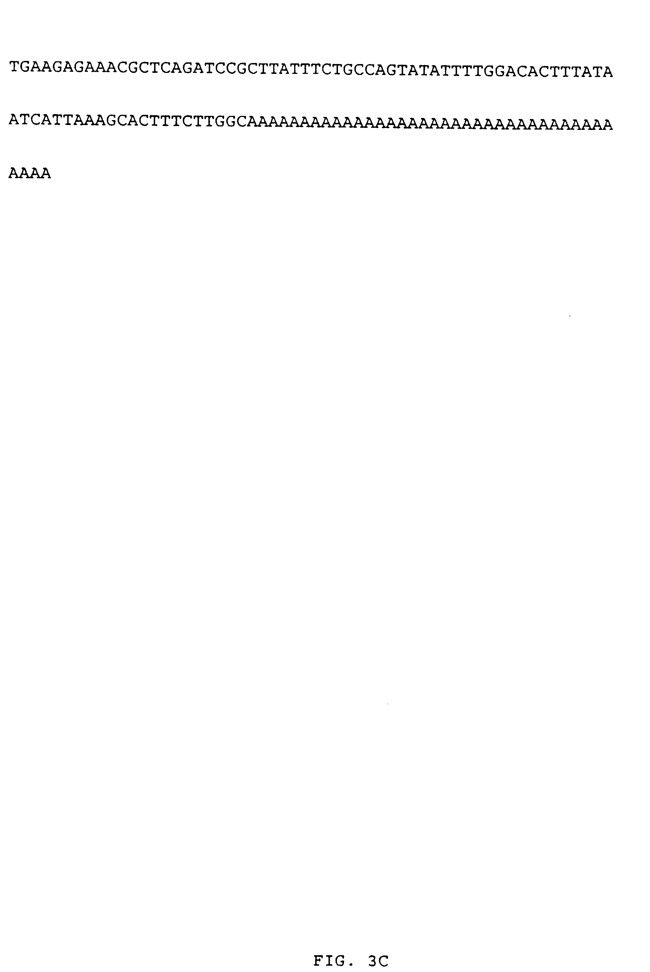

Further sequencing data was obtained by performing sequential exonuclease III deletions of the pGEM-7Zf(+) clone using a commercial deletion system (Erase-a-Base® system, Promega Corporation). Exonuclease III was used to specifically digest DNA from a 5' protruding or blunt end, while leaving a 4-base 3' protruding end intact. The uniform rate of digestion of the enzyme allowed deletions to be made at predetermined intervals by removing timed aliquots from the reaction. The strategy eventually produced substantially the entire nucleotide sequence of the cloned insert. Reference is made to Figs. 3-3C for an illustration of the nucleotide sequence of the cloned gene for human PRI obtained in this manner, which includes the derived amino acid sequence of the protein.

Given the nucleotide sequence of the cloned gene for human PRI, it would be within the scope of the present invention to construct a hybridization probe which would allow the isolation of the PRI gene by hybridization to a cDNA library.

### Example 6

### Engineering the Gene for Expression of the Natural Sequence Protein

To synthesize the natural sequence protein with no fusion partner attached to the gene product, it was assumed that the first ATG codon found in the sequencing on the clone was the natural translation start site. A prokaryotic ribosome binding site was then inserted 6 to 8 bases from the ATG start so that the start site would be functional in E.coli. From other constructions attempted, it appeared that PRI chimeric proteins with as few extra amino terminal amino acids as 8 were lethal when expressed from a regulatable E. coli promoter. It was therefore decided to drive the PRI gene with a T7 promoter, as described in Studier, F. William and Barbara A. Moffatt, "Use of Bacteriophage T7 RNA Polymerase to Direct Selective High-Level Expression of Cloned Genes," J. Mol. Biol., 189, 113-130 (1986).

The constructions were then completed and propagated in an E.coli host devoid of T7 RNA polymerase. Thus, the gene could be kept silent until transferred into a host expressing the RNA polymerase. Such expression systems have been described (Studier, et al., supra, and Rosenberg, Alan H., et al., "Vectors for Selective Expression of Cloned DNA's by T7 RNA Polymerase," Gene, 56, 125-135 (1987)) and were essentially followed in this example.

In a preferred construction, the ribosome binding site and spacing before the ATG codon, as well as the 5' untranslated leader which follows before the Shine-Dalgarno sequence, derive from the bacteriophage T7 gene 10 leader and ribosome binding site. In addition, a T7 promoter is present in order to drive production of this RNA.

The transfer of the PRI gene from lambda gtll into the pGEM®-7Zf(+) plasmid opened up a variety of cloning strategies due to the fact that the gene was now flanked by multiple cloning restriction enzyme sites present in the plasmid.

The PRI gene was excised on a Bam Hl-Aat II fragment and placed into a plasmid vector pBR322 between these two sites. A T7 terminator of the sequence referred to in Rosenberg, et al. (supra) and synthesized as a synthetic oligonucleotide, was then inserted after the gene between the Xba I and Aat II sites. The Xba I site was derived from the pGEM®-7ZF(+) linker upon transfer of the PRI gene on the Bam HI-Aat II fragment.

A synthetic oligonucleotide was synthesized that carried the T7 promoter and the gene 10 5' untranslated sequence and the gene 10 Shine-Dalgarno sequence.

The nucleotide was inserted into the beginning of the PRI gene between the Bam HI and the BstXI sites of the clone. The sequence analysis of the beginning of the PRI insert revealed a BstXI site after the third codon of the presumptive gene sequence. The oligonucleotide was designed to replace those amino acids since BstXI cuts just into the coding sequence. The final construct is illustrated in Fig. 4 and constitutes the PRI gene in the plasmid pBR322 engineered for expression in E. coli.

To express the PRI gene in this synthetic construct, the plasmid diagrammed in Fig. 4 was placed into an E. coli strain which produces T7 RNA polymerase. The strain was constructed according to the methods described in Studier, et al. (supra) and Rosenburg, et al. (supra). An E.coli strain JM 109 was lysogenized with wild type phage lambda. This strain was used as a host for infection by a recombinant lambda phage (lambda D69-T7) carrying the gene for T7 RNA polymerase inserted into the Bam HI site of the lambda vector. The gene for the T7 RNA polymerase was inserted into the chromosome of the E. coli strain JM109-DE3. A deposit of the clone was made with the American Type Culture Collection, 12301 Parklawn Drive, Rockville, Maryland 20850, on February 21, 1990 and received accession number 68230.

A culture was grown to an optical density of 1 and induced with IPTG. The PRI protein which was produced was monitored by polyacrylamide gel electrophoresis (PAGE) (Laemmli, U.K., Nature (London), 227, 680 (1970) of the bacteria proteins followed by Western blotting (Towbin, H., et al., supra.) and detection using primary rabbit antibody against PRI protein. The analysis indicated that following induction of the PRI culture with IPTG and growth for 4 hours, about 40 mgs. of PRI was synthesized per liter of culture.

### Example 7

### Production of Active PRI in E. coli JM 109

E. coli JM 109-DE3, carrying the PRI plasmid described in Example 6, can produce a certain level of active PRI protein. Although most of the protein is synthesized in an inactive and insoluble form, about 0.1% of the total PRI protein is both soluble and active. The active PRI protein may be purified from centrifuged French pressed lysates (Scopes, Robert K., Protein Purification-Principles and Practice, 2nd ed., 27, 1987) of this strain using batch absorption onto an affinity matrix containing bound RNase. The PRI recovered by this procedure has the same molecular weight on gels as natural PRI, a non-glycosylated protein described in Blackburn, et al. (supra) and Blackburn (supra), and about the same specific activity as the purified natural product.

Because most of the PRI protein produced in E.coli was synthesized in an insoluble form, to obtain an economical way of producing the active recombinant PRI protein, the insoluble material first had to be solubilized and refolded into an active form.

### Example 8

### Production of PRI Protein Under the Control of the E. coli Tac Promoter

The natural PRI protein was also produced in E. coli under the control of the strong host promoter pTac in a pTTQ vector (Stark, J.J.R., Gene, 51, pp. 255-267, 1987). The PRI insert was removed from pBR322 on an Sph I-Eco R1 fragment, which contains the good ribosome binding site and precedes the PRI gene, and cloned into the vector pTTQ19 (Amersham) between the SphI and Eco R1 sites. This construction enabled the PRI gene to be driven by the Tac promoter on a bicistronic message. One ribosome binding site is contributed by the pTTQ vector and the other by the PRI insert. Because internal ribosome binding sites function well in E. coli, the natural PRI protein, not a fusion protein, will be synthesized in this construct. The construct was placed into several different E. coli hosts in order to see which host gave the best levels of soluble and active protein. The hosts tested included E. coli C6000, JM109, NM522, BSJ 72, B121 and HB101. At least some soluble and active PRI was produced in each of the hosts tested.

### Example 9

### Secretion of PRI Protein in E. coli

The PRI protein might have a number of disulphide linkages as the amino acid analysis of the natural protein indicates 32 cysteines per molecule. These bonds might not form correctly in the reducing environment of the cytoplasm. Therefore, it was decided to attempt to secrete the protein into the bacterial periplasmic space where under more oxidizing conditions correct disulphide bond formation might take place. pBR322-PRI was modified by the addition of a synthetic oligonucleotide coding for the signal sequence of the E.coli ompA protein (Movva, N.R., et al., J. Mol. Biol., 143, pp. 317-238, 1980) placed before the start of the PRI gene. These leader amino acids target a protein for secretion and are cleaved off by the E. coli signal peptidase during secretion of the protein from the cell. In this construct the T7 promoter continues to drive transcription of the PRI gene, as in pBR322-PRI, but the ompA signal sequence is made first. Results indicated that PRI was correctly processed (the signal sequence removed) and secreted from the cell. The secreted PRI protein was found not to be active. Furthermore, levels of protein were about 10 fold lower than that produced inside the cell, amounting to only about 4 mg per liter of culture.

### Example 10

### Examples of PRI Constructs that are Lethal to E. coli

During the course of the construction of a useful expression vector for human recombinant PRI, several situations were encountered in which the expression of the PRI protein was not possible because these constructs proved to be lethal to the host cell. One such case was the attempted construction of a PRI fusion with twelve extra amino terminal amino acids in the vector pTTQ9, and another the secretion of PRI in the secretion expression vector PINIIIompA (Masui, Y., et al., "Multipurpose Expression Cloning Vehicles in Escherichia coli," Experimental Manipulation of Gene Expression, M. Inouye, Ed., Academic Press, 1983). In the first case, the Pst I site of pTTQ9 was first cut with Pst I, the ends blunted with Klenow, and the vector religated. The PRI insert from pGEM®-7Zf(+)-PRI was then removed on a Bam H1-Sph I fragment and cloned between the Bam H1 and Eco R1 sites of the Pst I filled pTTQ9 vector. Cutting then with Sal I and Kpn I followed by Klenow treatment of these sticky ends and religation would have put the PRI gene in frame for fusion protein expression from the strong Tac promoter on the vector. Since it was not possible to obtain the final constuct, it was concluded that this expression system cause lethal amounts of PRI fusion protein to be produced, even under uninduced conditions. It was later determined that this same fusion protein could be produced under the control of a T7 promoter, in essentially the same construct as pBR322-PRI, but this time with the clone engineered at the amino terminus to incorporate the extra twelve amino acids. The difference in the ability to produce the fusion protein in one expression system but not in the other is perhaps due to the greater repression of the system under conditions of non-induction.

In the attempt to clone the PRI insert into the secretion vector pIN IIIompA, lethality was also observed. The PRI insert was excised from pGEM®-7Zf(+)-PRI on the BstX1-EcoR1 fragment and cloned into EcoRl cut phosphatased pIN IIIompA. The PRI fragment could be inserted in two different orientations, directed so that the PRI gene is forwards or backwards with respect to the Tac promoter on the vector. The only observed constructs, however, were those in which the PRI insert was oriented backwards with respect to this promoter, and thus no expression of the gene was obtained. The forwards construct appears to be lethal, for reasons alluded to above.

### Example 11

### Isolation and Recovery of Insoluble PRI Produced in E. coli

The insoluble PRI produced in E. coli cells induced for production of this protein can be recovered as follows:
1. The cells induced for the production of the inactive PRI are suspended in a buffer solution. A suitable buffer solution is TESDTT buffer including 50 mM Tris-HCl (pH 7.5), 5 mM EDTA and 5 MM DTT. A suitable suspension includes approximately 20 grams of the cells, generally in a frozen state, in 400 ml. of the buffer solution. It is within the scope of this invention to scale the process up or down. However, it is preferred that a 1:20 ratio (gram cells/ml. buffer) be maintained.
2. The suspension is stirred vigorously for approximately 10 minutes on ice.
3. The cell membranes are then disrupted by cell lysis. A preferred cell lysis method includes adding powdered lysozyme (SIGMA grade VI) at a concentration of 1 mg/ml followed by stirring for 30 minutes on ice. After stirring, approximately 4 ml of 10% (v/v) sodium dodecyl sulfate (SDS) is added, again followed by 30 minutes of stirring on ice.
4. The suspension is then centrifuged at, preferably, about 17,000 X g for approximately 20 minutes at a temperature of about 4°C. A biphasic pellet will be recovered consisting of a greyish hard lower layer, a translucent slimy layer and supernatant.
5. The supernatant is decanted from the pellet carefully leaving the pellet intact. The pellet is the crude PRI protein.

### Example 12

### Purification of PRI

1. The crude PRI is isolated by resuspending the pellet obtained as in Example 11 in a buffer under similar conditions as in Example 11.
2. The suspension is then recentrifuged at approximately 17,000 X g for approximately 20 minutes at a temperature of about 4° C. After centrifugation, much less of the slimy layer will be present.
3. The supernatant is then decanted from the centrifuged mixture.
4. The pellet is resuspended in a buffer solution with stirring. Preferably, the buffer solution is TE5DTT in an amount of 400 ml. per 20 grams of pellet. The suspension is stirred vigorously for approximately 30 minutes on ice.
5. The suspension is then recentrifuged at preferably 17,000 X g for about 20 minutes at a temperature of about 4° C. The remaining pellet will be slightly grey in color.

### Example 13

### Solubilization of PRI

The pellet is resuspended and solubilized in the following manner:
1. The final pellet is resuspended in a buffer solution, preferably 200 ml. TE5DTT, with stirring for approximately 120 minutes on ice. A white milky suspension will result.
2. Solubilization is initiated in a buffer solution preferably containing urea in a concentration no less than 4M. A suitable buffer solution is TE5DTT. The urea solution preferably includes 800 ml. TE5DTT containing 360 g. urea.
3. The solubilized PRI is diluted at a rate of approximately 200 ml. PRI to 800 ml. buffer solution at a temperature between about 10° C and 25° C with rapid stirring. After the solution has been stirred for about 3 to 5 minutes, the solution should become almost clear with some debris.
4. Prior to use, the urea solution is preferably centrifuged to remove undissolved material. Centrifugation is accomplished at 6,000 X g for approximately 15 minutes at a temperature of about 4° C. A supernatant results, which is clear and colorless. The supernatant contains the solubilized PRI.

### Example 14

### Activation of PRI

The supernatant is rapidly diluted into 100 L of a buffer solution for a time sufficient to allow the PRI to refold to an active state. Preferably, the buffer solution is TE5DTT including 10% (v/v) of a water active agent. A preferred water active agent is glycerol, although sucrose may be substituted. The buffer solution has a pH between about 6.5 and about 8.5, preferably 7.5.

After the solution has allowed to sit for approximately 8 to 18 hours at room temperature, the PRI protein will have refolded such that about 7% of the molecules will become active.

### Example 15

### Separation of Refolded and Inactive PRI

The refolded PRI in the 100 L volume can be concentrated on a diethyl amino ethyl (DEAE) column reactor. The DEAE column is advantageous in that it not only concentrates the refolded PRI but also has been found to assist in completing the refolding process of the PRI protein, which is of significant advantage to the present invention. An exemplary column is a Cuno® 3200 DEAE cartridge at a flow rate of 30 liters/hour. The procedure is as follows:
1. The refolded PRI is pumped onto the cartridge and eluted with TE5DTT which also contains 0.5 M Nacl.
2. The eluate from the column is then applied to an affinity resin containing at least 1 mg/ml covalently bound RNase. The active PRI sticks to the RNase column while the inactive improperly folded material flows through in the affinity column fraction.
3. The active PRI is eluted from the affinity resin with 0.05 M Na-acetate buffer, pH 5.0 + 3M NaCl also containing 5 mM DTT. At this stage, the PRI is essentially pure and is homogeneous by PAGE.
4. If RNase contamination is a problem, the PRI protein may be further cleaned away from functional impurities by binding to and gradient elution from a DEAE Sepharose* CL-6B. The yield of PRI from 20 grams of cells by refolding and purification steps is about three million units.

*Sepharose is believed to be a registered trade march in one or more of the designated states.

The recombinant protein can be distinguished from the natural protein by the presence of an unblocked amino terminus on the recombinant protein. We have found that the natural source PRI cannot be sequenced from its amino end because its amino terminus is blocked, presumably by acetylation. Protein sequencing of the recombinant protein demonstrates an unblocked amino terminus for the recombinant, and furthermore indicates that the N terminal methionine has been removed in E. coli.

It is understood that the invention is not limited to the particular embodiments specifically disclosed in the Examples.

## Claims (Claims for the following Contracting State(s): AT, BE, CH, LI, DE, DK, FR, GB, IT, LU, NL, SE)

1. A plasmid which is replicatable in E. coli and comprises a DNA sequence encoding human placental ribonuclease inhibitor in a DNA segment which is the same as the segment of plasmid pBR322-PRI that corresponds to the sequence encoding human placental ribonuclease inhibitor that lies between the destroyed BamHI and destroyed AatII sites as shown in Figure 4.

2. A plasmid according to claim 1 which is plasmid pBR322-PRI.

3. An E. coli strain which comprises an inducible T7 RNA polymerase gene and is transformed with a plasmid as claimed in claim 1 or 2.

4. A strain according to claim 3 which is a transformed JM109-DE3 strain.

5. A method of obtaining cloned, active human placental ribonuclease inhibitor, which method comprises culturing an E. coli strain as defined in claim 3 or 4 and isolating, in active form, human placental ribonuclease inhibitor thus produced.

6. A method of solubilizing and activating human placental ribonuclease inhibitor from inclusion bodies produced in E. coli, which method comprises:
combining the inclusion bodies with a first buffer solution comprising urea at a concentration no less than 4M over a period of time no greater than thirty minutes;
combining the solution of inactive inhibitor with a second buffer solution at a rate of at least 20 parts second buffer to 1 part of the solution of inactive inhibitor, said second buffer having a pH between 6.5 and 8.5, comprising DTT at a concentration of not less than 0.5 mM, and EDTA; and
allowing sufficient time for the inhibitor to refold to an active state.

7. A method according to claim 6 wherein the first buffer solution has a pH of about 7.5 and further comprises EDTA at a concentration of about 5 mM and DTT at a concentration of about 0.5 mM.

8. A method according to claim 7 wherein the first buffer solution is Tris-HCl.

9. A method according to claims 6, 7 or 8 wherein the inclusion bodies and first buffer solution are combined at a temperature between 10°C and 25°C.

10. A method according to claim 9 wherein the inclusion bodies and first buffer solution are combined with rapid stirring.

11. A method according to anyone of claims 6 to 10 wherein the second buffer solution further comprises sucrose or glycerol at a concentration of about 10% (v/v).

12. A method according to anyone of claims 6 to 11 wherein the second buffer solution is Tris-HCl.

13. A method according to anyone of claim 6 to 12 wherein the EDTA concentration in the second buffer solution is about 5 mM.

14. A method according to claim 13 wherein the DTT concentration in the second buffer solution is about 0.5 mM.

15. A method of solubilizing and refolding human placental ribonuclease inhibitor produced by a recombinant host cell comprising:
a) isolating inactive human placental ribonuclease inhibitor produced by the host cell; and
b) solubilizing and activating the inactive human placental ribonuclease inhibitor by a method in accordance with any one of claims 6 to 14.

16. A method of obtaining cloned, active placental ribonuclease inhibitor, which method comprises culturing an E. coli strain as claimed in claim 3 or 4 and isolating, solubilizing and activating human placental ribonuclease inhibitor thus produced in accordance with claim 15.

## Claims (Claims for the following Contracting State(s): ES)

1. A process for producing a plasmid which is replicatable in E. coli and comprises a DNA sequence encoding human placental ribonuclease inhibitor in a DNA segment which is the same as the segment of plasmid pBR322-PRI that corresponds to the sequence encoding human placental ribonuclease inhibitor that lies between the destroyed BamHI and destroyed AatII sites as shown in Figure 4, which process comprises subcloning the DNA encoding human placental ribonuclease inhibitor into a plasmid which is replicatable in E. coli such that a gene product consisting of human placental ribonuclease inhibitor will be expressed.

2. A process according to claim 1 wherein the plasmid produced is pBR322-PRI.

3. A process for producing an E. coli strain which is capable of producing human placental ribonuclease inhibitor and which comprises an inducible T7 RNA polymerase gene which process comprises transforming E. coli with a plasmid which is replicatable in E. coli and comprises a DNA sequence encoding human placental ribonuclease inhibitor in a DNA segment which is the same as the segment or plasmid pBR322-PRI that lies between the destroyed BamHI and destroyed AatII sites as shown in Figure 4.

4. A process according to claim 3 wherein the strain is a transformed JM109-DE3 strain and the plasmid is pBR322-PRI.

5. A method of obtaining cloned, active human placental ribonuclease inhibitor, which method comprises culturing an E. Coli strain as defined in claim 3 or 4 and isolating, in active form, human placental ribonuclease inhibitor thus produced.

6. A method of solubilizing and activating human placental ribonuclease inhibitor from inclusion bodies produced in E. coli, which method comprises:
combining the inclusion bodies with a first buffer solution comprising urea at a concentration no less than 4M over a period of time no greater than thirty minutes;
combining the solution of inactive inhibitor with a second buffer solution at a rate of at least 20 parts second buffer to 1 part of the solution of inactive inhibitor, said second buffer having a pH between 6.5 and 8.5, comprising DTT at a concentration of not less than 0.5 mM, and EDTA; and
allowing sufficient time for the inhibitor to refold to an active state.

7. A method according to claim 6 wherein the first buffer solution has a pH of about 7.5 and further comprises EDTA at a concentration of about 5 mM and DTT at a concentration of about 0.5 mM.

8. A method according to claim 7 wherein the first buffer solution is Tris-HCl.

9. A method according to claims 6, 7 or 8 wherein the inclusion bodies and first buffer solution are combined at a temperature between 10°C and 25°C.

10. A method according to claim 9 wherein the inclusion bodies and first buffer solution are combined with rapid stirring.

11. A method according to any one of claims 6 to 10 wherein the second buffer solution further comprises sucrose or glycerol at a concentration of about 10% (v/v).

12. A method according to any one of claims 6 to 11 wherein the second buffer solution is Tris-HCl.

13. A method according to any one of claims 6 to 12 wherein the EDTA concentration in the second buffer solution is about 5 mM.

14. A method according to claim 13 wherein the DTT concentration in the second buffer solution is about 0.5 mM.

15. A method of solubilizing and refolding human placental ribonuclease inhibitor produced by a recombinant host cell comprising:
a) isolating inactive human placental ribonuclease inhibitor produced by the host cell; and
b) solubilizing and activating the inactive human placental ribonuclease inhibitor by a method in accordance with any one of claims 6 to 14.

16. A method of obtaining cloned, active placental ribonuclease inhibitor, which method comprises culturing an E. coli strain as claimed in claim 3 or 4 and isolating, solubilizing and activating human placental ribonuclease inhibitor thus produced in accordance with claim 15.

17. A plasmid which is replicatable in E. coli and comprises a DNA sequence encoding human placental ribonuclease inhibitor in a DNA segment which is the same as the segment of plasmid pBR322-PRI that corresponds to the sequence encoding human placental inhibitor that lies between the destroyed BamHI and destroyed AatII sites as shown in Figure 4.

18. An E. coli strain which comprises an inducible T7 RNA polymerase gene and is transformed with a plasmid which is replicatable in E. coli and comprises a DNA sequence encoding human placental ribonuclease inhibitor in a DNA segment which is the same as the segment of plasmid pBR322-PRI that corresponds to the sequence encoding human placental inhibitor that lies between the destroyed BamHI and destroyed AatII sites as shown in Figure 4.

## Patentansprüche (Patentansprüche für folgende(n) Vertragsstaat(en): AT, BE, CH, LI, DE, DK, FR, GB, IT, LU, NL, SE)

1. Plasmid, das in E. coli replizierbar ist und eine DNA-Sequenz enthält, die einen menschlichen, plazentalen Ribonuclease-Inhibitor in einem DNA-Segment codiert, das dasselbe ist wie das Segment des Plasmids pBR322-PRI, das der Sequenz entspricht, die einen menschlichen, plazentalen Ribonuclease-Inhibitor codiert, der zwischen den zerstörten BamHI-und AatII-Stellen liegt, wie in Figur 4 dargestellt.

2. Plasmid gemäß Anspruch 1, wobei das Plasmid pBR322-PRI ist.

3. E. coli-Stamm der ein induzierbares T7-RNA-Polymerase-Gen enthält und mit einem Plasmid gemäß Anspruch 1 oder 2 tranformiert ist.

4. Stamm gemäß Anspruch 3, der ein transformierter JM109-DE3-Stamm ist.

5. Verfahren zum Erhalt eines geklonten, aktiven menschlichen plazentalen Ribonuclease-Inhibitors, wobei das Verfahren die Kultur eines in Anspruch 3 oder 4 definierten E. coli-Stammes umfaßt und die Isolierung, in aktiver Form, des so erzeugten menschlichen plazentalen Ribonuclease-Inhibitors.

6. Verfahren der Solubilisierung und Aktivierung eines menschlichen plazentalen Ribonuclease-Inhibitors aus in E. coli produzierten Einschlußkörpern, wobei das Verfahren die Schritte umfaßt:
Kombinieren der Einschlußkörper mit einer ersten Pufferlösung, enthaltend Harnstoff bei einer Konzentration von nicht weniger als 4 M, über einen Zeitraum von nicht größer als 30 Minuten;
Kombinieren der Lösung von inaktivem Inhibitor mit einer zweiten Pufferlösung bei einer Rate von wenigstens 20 Teilen des zweiten Puffers zu 1 Teil der Lösung des inaktiven Inhibitors, wobei der zweite Puffer einen pH zwischen 6,5 und 8,5 besitzt, DTT bei einer Konzentration von nicht weniger als 0,5 mM aufweist und EDTA; und
zur Verfügung stellen von ausreichend Zeit für den Inhibitor zum Entfalten in einen aktiven Zustand.

7. Verfahren gemäß Anspruch 6, bei dem die erste Pufferlösung einen pH von ungefähr 7,5 besitzt und ferner EDTA in einer Konzentration von ungefähr 5 mM und DTT in einer Konzentration von ungefähr 0,5 mM enthält.

8. Verfahren gemäß Anspruch 7, bei dem die erste Pufferlösung Tris-HCl ist.

9. Verfahren gemäß den Ansprüchen 6, 7 oder 8, bei dem die Einschlußkörper und die erste Pufferlösung bei einer Temperatur zwischen 10° Celsius und 25° Celsius vereinigt werden.

10. Verfahren gemäß Anspruch 9, bei dem die Einschlußkörper und die erste Pufferlösung unter schnellem Rühren vereinigt werden.

11. Verfahren gemäß einem der Ansprüche 6 bis 10, bei dem die zweite Pufferlösung ferner Sucrose oder Glycerol in einer Konzentration von ungefähr 10% (v/v) enthält.

12. Verfahren gemäß einem der Ansprüche 6 bis 11, bei dem die zweite Pufferlösung Tris-HCl ist.

13. Verfahren gemäß einem der Ansprüche 6 bis 12, bei dem die EDTA-Konzentration in der zweiten Pufferlösung ungefähr 5 mM ist.

14. Verfahren gemäß Anspruch 13, bei dem die DTT-Konzentration in der zweiten Pufferlösung ungefähr 0,5 mM ist.

15. Verfahren des Solubilisierens und Entfaltens eines menschlichen plazentalen Ribonuclease-Inhibitors, der durch eine rekombinante Wirtszelle erzeugt wurde, wobei das Verfahren folgende Schritte aufweist:
a) Isolieren eines inaktiven, menschlichen plazentalen Ribonuclease-Inhibitors, der durch die Wirtszelle erzeugt wurde; und
b) Solubilisieren und Aktivieren des inaktiven menschlichen plazentalen Ribonuclease-Inhibitors durch ein Verfahren gemäß einem der Ansprüche 6 bis 14.

16. Verfahren zum Erhalt eines geklonten, aktiven plazentalen Ribonuclease-Inhibitors, wobei das Verfahren die Kultur eines E. coli-Stammes gemäß Anspruch 3 oder 4 und ein Isolieren, Solubilisieren und Aktivieren des so gemäß Anspruch 15 erzeugten menschlichen plazentalen Ribonuclease-Inhibitors umfaßt.

## Patentansprüche (Patentansprüche für folgende(n) Vertragsstaat(en): ES)

1. Verfahren zur Herstellung eines Plasmids, das in E. coli replizierbar ist und eine DNA-Sequenz enthält, die einen menschlichen plazentalen Ribonuclease-Inhibitor in einem DNA-Segment codiert, das dasselbe ist wie das Segment des Plasmids pBR322-TRI, das der Sequenz entspricht, die einen menschlichen plazentalen Ribonuclease-Inhibitor codiert, die zwischen den zerstörten BamHI und AatII-Stellen liegt, wie in Figur 4 dargestellt, wobei das Verfahren ein Subklonieren der DNA, die einen menschlichen plazentalen Ribonuclease-Inhibitor codiert, in ein Plasmid umfaßt, das in E. coli replizierbar ist, so daß ein Genprodukt bestehend aus menschlichem plazentalen Ribonuclease-Inhibitor exprimiert wird.

2. Verfahren gemäß Anspruch 1, bei dem das erzeugte Plasmid pBR322-PRI ist.

3. Verfahren zur Herstellung eines E. coli-Stammes der zur Produktion eines menschlichen plazentalen Ribonuclease-Inhibitors fähig ist und der ein induzierbares T7-RNA-Polymerase-Gen enthält, wobei das Verfahren ein Transformieren von E. coli mit einem Plasmid, das in E. coli replizierbar ist und eine DNA-Sequenz enthält, die einen menschlichen plazentalen Ribonuclease-Inhibitor in einem DNA-Segment codiert, das dasselbe ist wie das Segment oder Plasmid pBR322-PRI, das zwischen den zerstörten BamHI- und AatII-Stellen liegt, wie in Figur 4 gezeigt, umfaßt.

4. Verfahren gemäß Anspruch 3, bei dem der Stamm ein transformierter JM109-DE3-Stamm ist und das Plasmid pBR322-PRI.

5. Verfahren zum Erhalt eines geklonten aktiven menschlichen plazentalen Ribonuclease-Inhibitors, wobei das Verfahren die Kultur eines E. coli-Stammes gemäß Anspruch 3 oder 4 und das Isolieren, in aktiver Form, von dem so erzeugten menschlichen plazentalen Ribonuclease-Inhibitor umfaßt.

6. Verfahren der Solubilisierung und Aktivierung eines menschlichen plazentalen Ribonuclease-Inhibitor aus in E. coli produzierten Einschlußkörpern, wobei das Verfahren die Schritte umfaßt:
Kombinieren der Einschlußkörper mit einer ersten Pufferlösung, enthaltend Harnstoff bei einer Konzentration von nicht weniger als 4 M, über einen Zeitraum von nicht größer als 30 Minuten;
Kombinieren der Lösung von inaktivem Inhibitor mit einer zweiten Pufferlösung bei einer Rate von wenigstens 20 Teilen des zweiten Puffers zu 1 Teil der Lösung des inaktiven Inhibitors, wobei der zweite Puffer einen pH zwischen 6,5 und 8,5 besitzt, DTT bei einer Konzentration von nicht weniger als 0,5 mM aufweist und EDTA; und
zur Verfügung stellen von ausreichend Zeit für den Inhibitor zum Entfalten in einen aktiven Zustand.

7. Verfahren gemäß Anspruch 6, bei dem die erste Pufferlösung einen pH von ungefähr 7,5 besitzt und ferner EDTA in einer Konzentration von ungefähr 5 mM und DTT in einer Konzentration von ungefähr 0,5 mM enthält.

8. Verfahren gemäß Anspruch 7, bei dem die erste Pufferlösung Tris-HCl ist.

9. Verfahren gemäß den Ansprüchen 6, 7 oder 8, bei dem die Einschlußkörper und die erste Pufferlösung bei einer Temperatur zwischen 10° Celsius und 25° Celsius vereinigt werden.

10. Verfahren gemäß Anspruch 9, bei dem die Einschlußkörper und die erste Pufferlösung unter schnellem Rühren vereinigt werden.

11. Verfahren gemäß einem der Ansprüche 6 bis 10, bei dem die zweite Pufferlösung ferner Sucrose oder Glycerol in einer Konzentration von ungefähr 10% (v/v) enthält.

12. Verfahren gemäß einem der Ansprüche 6 bis 11, bei dem die zweite Pufferlösung Tris-HCl ist.

13. Verfahren gemäß einem der Ansprüche 6 bis 12, bei dem die EDTA-Konzentration in der zweiten Pufferlösung ungefähr 5 mM ist.

14. Verfahren gemäß Anspruch 13, bei dem die DTT-Konzentration in der zweiten Pufferlösung ungefähr 0,5 mM ist.

15. Verfahren des Solubilisierens und Entfaltens eines menschlichen plazentalen Ribonuclease-Inhibitors, der durch eine rekombinante Wirtszelle erzeugt wurde, wobei das Verfahren folgenden Schritte aufweist:
a) Isolieren eines inaktiven, menschlichen plazentalen Ribonuclease-Inhibitors, der durch die Wirtszelle erzeugt wurde; und
b) Solubilisieren und Aktivieren des inaktiven menschlichen plazentalen Ribonuclease-Inhibitors durch ein Verfahren gemäß einem der Ansprüche 6 bis 14.

16. Verfahren zum Erhalt eines geklonten, aktiven plazentalen Ribonuclease-Inhibitors, wobei das Verfahren die Kultur eines E. coli-Stammes gemäß Anspruch 3 oder 4 und ein Isolieren, Solubilisieren und Aktivieren des so gemäß Anspruch 15 erzeugten menschlichen plazentalen Ribonuclease-Inhibitors umfaßt.

17. Plasmid, das in E. coli replizierbar ist und eine DNA-Sequenz enthält, die menschlichen plazentalen Ribonuclease-Inhibitor in einem DNA-Segment codiert, das dasselbe wie das Segment von Plasmid pBR322-PRI ist, das der Sequenz entspricht, die menschlichen plazentalen Ribonuclease-Inhibitor codiert, wobei die Sequenz zwischen zerstörten BamHI- und AaTII-Stellen liegt, wie in Figur 4 dargestellt.

18. E. coli-Stamm, der ein induzierbares T7-RNA-Polymerase-Gen enthält und mit einem Plasmid transformiert ist, das in E. coli replizierbar ist und eine DNA-Sequenz enthält, die menschlichen plazentalen Ribonuclease-Inhibitor in einem DNA-Segment codiert, das dasselbe wie das Segment pBR322-PRI ist, das der Sequenz entspricht die menschlichen plazentalen Inhibitor codiert, wobei die Sequenz zwischen den zerstörten BamHI- und AatII-Stellen liegt, wie in Figur 4 dargestellt.

## Revendications (Revendications pour l'(les) Etat(s) contractant(s) suivant(s): AT, BE, CH, LI, DE, DK, FR, GB, IT, LU, NL, SE)

1. Plasmide qui est réplicable dans E.coli et comprend une séquence d'ADN codant pour un inhibiteur de ribonucléase placentaire humain dans un segment d'ADN qui est le même que le segment du plasmide pBR322-PRI qui correspond à la séquence codant pour l'inhibiteur de ribonucléase placentaire humain qui s'étend entre les sites BamHI détruit et AatII détruit comme représenté à la figure 4.

2. Plasmide selon la revendication 1, qui est le plasmide pBR322-PRI.

3. Souche d'E.coli qui comprend un gène d'ARN polymérase T7 inductible et est transformée avec un plasmide tel que revendiqué dans la revendication 1 ou 2.

4. Souche selon la revendication 3, qui est une souche JM109-DE3 transformée.

5. Méthode d'obtention d'inhibiteur de ribonucléase placentaire humain actif, cloné, méthode qui comprend les étapes consistant à cultiver une souche d'E.coli telle que définie à la revendication 3 ou 4 et à isoler, sous une forme active, l'inhibiteur de la ribonucléase placentaire humain ainsi produit.

6. Méthode de solubilisation et d'activation d'inhibiteur de ribonucléase placentaire humain à partir de corps d'inclusion produits dans E.coli, méthode qui comprend les étapes consistant:
à combiner les corps d'inclusion avec une première solution tampon comprenant de l'urée à une concentration non inférieure à 4 M pendant une période de temps non supérieure à 30 minutes;
à combiner la solution d'inhibiteur inactif avec une seconde solution tampon à un taux d'au moins 20 parties de second tampon pour 1 partie de la solution d'inhibiteur inactif, ledit second tampon ayant un pH entre 6,5 et 8,5, comprenant du DTT à une concentration non inférieure à 0,5 mM, et de l'EDTA; et
à laisser un temps suffisant pour que l'inhibiteur se replie en un état actif.

7. Méthode selon la revendication 6, dans laquelle la première solution tampon a un pH d'environ 7,5 et comprend, en outre, de l'EDTA à une concentration d'environ 5 mM et du DTT à une concentration d'environ 0,5 mM.

8. Méthode selon la revendication 7, dans laquelle la première solution tampon du Tris-HCl.

9. Méthode selon les revendications 6, 7 ou 8, dans laquelle les corps d'inclusion et la première solution tampon sont combinés à une température entre 10°C et 25°C.

10. Méthode selon la revendication 9, dans laquelle les corps d'inclusion et la première solution tampon sont combinés avec agitation rapide.

11. Méthode selon l'une quelconque des revendications 6 à 10, dans laquelle la seconde solution tampon comprend, en outre, du saccharose ou du glycérol à une concentration d'environ 10% (v/v).

12. Méthode selon l'une quelconque des revendications 6 à 11, dans laquelle la seconde solution tampon est du
Tris-HCl.

13. Méthode selon l'une quelconque des revendications 6 à 12, dans laquelle la concentration d'EDTA dans la seconde solution tampon est d'environ 5 mM.

14. Méthode selon la revendication 13, dans laquelle la concentration de DTT dans la seconde solution tampon est d'environ 0,5 mM.

15. Méthode de solubilisation et de repliage d'inhibiteur de ribonucléase placentaire humain produit par une cellule hôte recombinante comprenant les étapes consistant:
a) à isoler l'inhibiteur de ribonucléase placentaire humain inactif produit par la cellule hôte; et
b) à solubiliser et à activer l'inhibiteur de ribonucléase placentaire humain inactif par une méthode selon l'une quelconque des revendications 6 à 14.

16. Méthode d'obtention d'inhibiteur de ribonucléase placentaire actif, cloné, méthode qui comprend les étapes consistant à cultiver une souche d'E.coli telle que revendiquée à la revendication 3 ou 4 et à isoler, à solubiliser et à activer l'inhibiteur de ribonucléase placentaire humain ainsi produit conformément à la revendication 15.

## Revendications (Revendications pour l'(les) Etat(s) contractant(s) suivant(s): ES)

1. Procédé pour produire un plasmide qui est réplicable dans E.coli et comprend une séquence ADN codant pour un inhibiteur de ribonucléase placentaire humain dans un segment d'ADN qui est le même que le segment du plasmide pBR322-PRI qui correspond à la séquence codant pour l'inhibiteur de ribonucléase placentaire humain qui s'étend entre les sites BamHI détruit et AatII détruit comme représenté à la figure 4, procédé qui comprend les étapes consistant à sous-cloner l'ADN codant pour l'inhibiteur de ribonucléase placentaire humain dans un plasmide qui est réplicable dans E.coli tel qu'un produit de gène consistant en l'inhibiteur de ribonucléase placentaire humain soit exprimé.

2. Procédé selon la revendication 1, dans lequel le plasmide produit est pBR322-PRI.

3. Procédé pour produire une souche d'E.coli qui est capable de produire un inhibiteur de ribonucléase placentaire humain et qui comprend un gène d' ARN polymérase T7 inductible , procédé qui comprend les étapes consistant à transformer E.coli avec un plasmide qui est réplicable dans E.coli et comprend une séquence d'ADN codant pour l'inhibiteur de ribonucléase placentaire humain dans un segment d'ADN qui est le même que le segment ou plasmide pBR322-PRI qui s'étend entre les sites BamHI détruit et AatII détruit comme représenté à la figure 4.

4. Procédé selon la revendication 3, dans lequel la souche est une souche JM109-DE3 transformée et le plasmide est pBR322-PRI.

5. Méthode d'obtention d'inhibiteur de ribonucléase placentaire humain actif, cloné, méthode qui comprend les étapes consistant à cultiver une souche d' E.coli telle que définie à la revendication 3 ou 4 et à isoler, sous une forme active, l'inhibiteur de ribonucléase placentaire humain ainsi produit.

6. Méthode de solubilisation et d'activation d'inhibiteur de ribonucléase placentaire humain à partir de corps d'inclusion produits dans E.coli, méthode qui comprend les étapes consistant:
à combiner les corps d'inclusion avec une première solution tampon comprenant de l'urée à une concentration non inférieure à 4 M pendant une période de temps non supérieure à 30 minutes;
à combiner la solution d'inhibiteur inactif avec une seconde solution tampon à un taux d'au moins 20 parties du second tampon pour 1 partie de la solution d'inhibiteur inactif, ledit second tampon ayant un pH entre 6,5 et 8,5, comprenant du DTT à une concentration non inférieure à 0,5 mM, et de l'EDTA; et
à laisser un temps suffisant pour que l'inhibiteur se replie en un état actif.

7. Méthode selon la revendication 6, dans laquelle la première solution tampon a un pH d'environ 7,5 et comprend, en outre, de l'EDTA à une concentration d'environ 5 mM et du DTT à une concentration d'environ 0,5 mM.

8. Méthode selon la revendication 7, dans laquelle la première solution tampon est du Tris-HCl.

9. Méthode selon les revendications 6, 7 ou 8, dans laquelle les corps d'inclusion et la première solution tampon sont combinés à une température entre 10°C et 25°C.

10. Méthode selon la revendication 9, dans laquelle les corps d'inclusion et la première solution tampon sont combinés avec une agitation rapide.

11. Méthode selon l'une quelconque des revendications 6 à 10, dans laquelle la seconde solution tampon comprend, en outre, du saccharose ou du glycérol à une concentration d'environ 10% (v/v).

12. Méthode selon l'une quelconque des revendications 6 à 11, dans laquelle la seconde solution tampon est du Tris-HCl.

13. Méthode selon l'une quelconque des revendications 6 à 12, dans laquelle la concentration d'EDTA dans la seconde solution tampon est d'environ 5 mM.

14. Méthode selon la revendication 13, dans laquelle la concentration de DTT dans la seconde solution tampon est d'environ 0,5 mM.

15. Méthode de solubilisation et de repliage d'inhibiteur de ribonucléase placentaire humain produit par une cellule hôte recombinante comprenant les étapes consistant:
a) à isoler l'inhibiteur de ribonucléase placentaire humain inactif produit par la cellule hôte; et
b) à solubiliser et à activer l'inhibiteur de ribonucléase placentaire humain inactif par une méthode conformément à l'une quelconque des revendications 6 à 14.

16. Méthode d'obtention d'inhibiteur de ribonucléase placentaire actif, cloné, méthode qui comprend les étapes consistant à cultiver une souche d'E.coli comme revendiqué dans la revendication 3 ou 4 et à isoler, à solubiliser et à activer l'inhibiteur de ribonucléase placentaire humain ainsi produit conformément à la revendication 15.

17. Plasmide qui est réplicable dans E.coli et comprend une séquence d'ADN codant pour un inhibiteur de ribonucléase placentaire humain dans un segment d'ADN qui est le même que le segment du plasmide pBR322-PRI qui correspond à la séquence codant pour l'inhibiteur de ribonucléase placentaire humain qui s'étend entre les sites BamHI détruit et AatII détruit comme représenté à la figure 4.

18. Souche d'E.coli qui comprend un gène d' ARN polymérase T7 inductible et est transformée avec un plasmide qui est réplicable dans E.coli et comprend une séquence d'ADN codant pour un inhibiteur de ribonucléase placentaire humain dans un segment d'ADN qui est le même que le segment du plasmide pBR322-PRI qui correspond à la séquence codant pour l'inhibiteur de ribonucléase placentaire humain qui s'étend entre les sites BamHI détruit et AatII détruit comme représenté à la figure 4.
